(19) Europäisches Patentamt
European Patent Office
Office européen des brevets

(11) Veröffentlichungsnummer: **0 097 881**
**B1**

(12) # EUROPÄISCHE PATENTSCHRIFT

(45) Veröffentlichungstag der Patentschrift: **29.08.90**

(21) Anmeldenummer: **83105895.3**

(22) Anmeldetag: **16.06.83**

(51) Int. Cl.$^5$: **C 07 D 249/08,**
C 07 D 233/60,
C 07 D 405/06,
A 01 N 43/653, A 01 N 43/50

(54) **Substituierte Azolyl-ketone und -alkohole.**

(30) Priorität: **29.06.82 DE 3224129**

(43) Veröffentlichungstag der Anmeldung:
**11.01.84 Patentblatt 84/02**

(45) Bekanntmachung des Hinweises auf die Patenterteilung:
**29.08.90 Patentblatt 90/35**

(84) Benannte Vertragsstaaten:
**AT BE CH DE FR GB IT LI NL SE**

(56) Entgegenhaltungen:

| | |
|---|---|
| EP-A-0 015 639 | EP-A-0 032 200 |
| EP-A-0 016 323 | EP-A-0 079 006 |
| EP-A-0 021 327 | DE-A-2 805 227 |
| EP-A-0 031 911 | DE-A-3 028 337 |

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Krämer, Wolfgang, Dr.**
**Am Eckbusch 39/45**
**D-5600 Wuppertal 1 (DE)**
Erfinder: **Büchel, Karl-Heinz, Prof. Dr.**
**Dabringhausener Strasse 42**
**D-5093 Burscheid (DE)**
Erfinder: **Ditgens, Klaus, Dr.**
**Claudiusweg 7**
**D-5600 Wuppertal 1 (DE)**
Erfinder: **Elbe, Hans-Ludwig, Dr.**
**Dasnoeckel 59**
**D-5600 Wuppertal 1 (DE)**
Erfinder: **Jäger, Gerhard, Dr.**
**Gellertstrasse 18**
**D-5090 Leverkusen (DE)**
Erfinder: **Jautelat, Manfred, Dr.**
**Muellersbaum 28**
**D-5093 Burscheid (DE)**
Erfinder: **Lürssen, Klaus, Dr.**
**August-Kierspel Strasse 89**
**D-5060 Bergisch-Gladbach 2 (DE)**
Erfinder: **Reinecke, Paul, Dr.**
**Lessingstrasse 11**
**D-5090 Leverkusen 3 (DE)**

**EP 0 097 881 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue substituierte Azolylketone und -alkohole, mehrere Verfahren zu ihrer Herstellung sowie ihre Verwendung als Fungizide und Pflanzenwachstumsregulatoren.

Es ist bereits bekannt geworden, daß bestimmte Azolylpentanone, wie beispielsweise 1-(4-chlorphenyl)-4,4-dimethyl-2-(1,2,4-triazol-1-yl)-3-pentanon, gute fungizide Eigenschaften besitzen (vergleiche DE—A—27 34 426). Außerdem ist bekannt geworden, daß bestimmte Azolylbutanone und -ole, wie beispielsweise alkylierte 3,3-Dimethyl-4-fluor(chlor)-1-(1,2,4-triazol-1-yl)-2-butanone und -ole, gute fungizide und pflanzenwachstumsregulierende Eigenschaften aufweisen (vergleiche DE—A—29 51 164, EP—A—0 031 911, DE—A—29 51 163 und EP—A—0 032 200).

Die Wirkung all dieser Verbindungen ist jedoch in bestimmten Indikationsbereichen, insbesondere bei niedrigen Aufwandmengen und -konzentrationen, nicht immer ganz befriedigend.

Im übrigen sind auch aus der DE—A—2 805 227, der EP—A—0 015 639, der DE—A—3 028 337, der EP—A—0 079 006 und der EP—A—0 016 323 Azolyl-Derivate mit fungiziden Eigenschaften bekannt.

Es wurden neue substituierte Azolyl-ketone und -alkohole der allgemeinen Formel

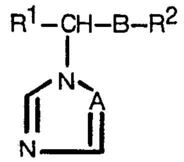

(I)

in welcher

A für ein Stickstoffatom oder die CH-Gruppe steht,

B für die Keto- oder CH(OH)-Gruppe steht,

$R^1$ für geradkettiges oder verzweigtes Alkyl mit 1 bis 12 Kohlenstoffatomen, für geradkettiges oder verzweigtes Alkinyl mit 2 bis 12 Kohlenstoffatomen, oder für gegebenenfalls einfach bis dreifach, gleich oder verschieden substituiertes Phenylalkyl mit 1 bis 4 Kohlenstoffatomen im Alkylteil steht, wobei als Phenylsubstituenten genannt seien;

Halogen; Alkyl, Alkoxy und Alkylthio mit jeweils 1 bis 4 Kohlenstoffatomen; Cyclohexyl; Dialkylamino mit 1 bis 4 Kohlenstoffatomen in jedem Alkylteil; Halogenalkyl, Halogenalkoxy und Halogenalkylthio mit jeweils 1 bis 4 Kohlenstoffatomen und 1 bis 5 gleichen oder verschiedenen Halogenatomen; Nitro; Cyano; Alkoxycarbonyl mit 1 bis 4 Kohlenstoffatomen im Alkoxyteil; sowie gegebenenfalls durch Halogen substituiertes Phenyl und Phenoxy; und ferner

$R^1$ für jeweils gegebenenfalls einfach bis dreifach, gleich oder verschieden durch Alkyl mit 1 bis 4 Kohlenstoffatomen substituiertes Cycloalkyl und Cycloalkylalkyl mit jeweils 3 bis 7 Kohlenstoffatomen im Cycloalkylteil und 1 bis 4 Kohlenstoffatomen im Alkylteil steht;

$R^2$ für einfach bis dreifach, gleich oder verschieden durch Alkyl mit 1 bis 4 Kohlenstoffatomen substituiertes Cycloalkyl mit 3 bis 7 Kohlenstoffatomen, sowie für die Gruppierung $—C(CH_3)_2R^3$ steht, wobei;

$R^3$ für geradkettiges oder verzweigtes Alkyl mit 3 bis 6 Kohlenstoffatomen, für geradkettiges oder verzweigtes Alkenyl mit 2 bis 4 Kohlenstoffatomen, für Alkinyl mit 3 bis 5 Kohlenstoffatomen oder die —CH=O-Gruppe, Alkoxyiminomethyl mit 1 bis 4 Kohlenstoffatomen im Alkoxyteil, Dialkoxymethyl mit 1 bis 4 Kohlenstoffatomen in jedem Alkoxyteil, Dioxolanyl und Dioxanyl steht,

sowie deren Säureadditions-Salze und Metallsalz-Komplexe gefunden.

Diejenigen Verbindungen der Formel (I), in denen B für eine CH(OH)-Gruppe steht, besitzen zwei asymmetrische Kohlenstoffatome; sie können deshalb in den beiden geometrischen Isomeren (erythro- und threo-Form) vorliegen, die in unterschiedlichen Mengenverhältnissen anfallen können. In allen Fällen liegen sie als Enantiomerenpaare vor.

Weiterhin wurde gefunden, daß man die neuen substituierten Azolyl-ketone und -alkohole der Formel (I) sowie deren Säureadditions-Salze und Metallsalz-komplexe erhält, wenn man

a) Azolyl-ketone der Formel

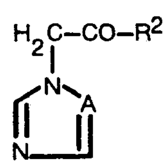

(II)

in welcher

A und $R^2$ die oben angegebene Bedeutung haben, mit einem Alkylierungsmittel der Formel

$$R^1—Z \qquad (III)$$

in welcher

$R^1$ die oben angegebene Bedeutung hat und

Z für eine elektronenanziehende Abgangsgruppierung steht,

in Gegenwart einer Base und in Gegenwart eines organischen Verdünnungsmittels, oder in einem wäßrigorganischen Zweiphasensystem in Gegenwart eines Phasentransferkatalysators, umsetzt; oder

b) Halogenketone der Formel

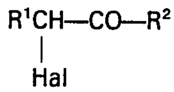

(IV)

in welcher

$R^1$ und $R^2$ die oben angegebene Bedeutung haben und

Hal für Chlor oder Brom steht,

mit Azolen der Formel

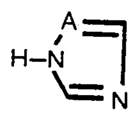

(V)

in welcher

die oben angegebene Bedeutung hat,

in Gegenwart eines Verdünnungsmittels und in Gegenwart eines Säurebindemittels umsetzt; und gegebenenfalls

c) die nach den Verfahren (a) und (b) erhaltenen Verbindungen der Formel

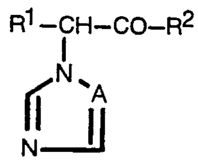

(Ia)

in welcher

A, $R^1$ und $R^2$ die oben angegebene Bedeutung haben,

nach bekannten Methoden in üblicher Weise reduziert und gegebenenfalls noch an die erhaltenen Verbindungen der Formel I anschließend eine Säure oder ein Metallsalz addiert.

Außerdem wurde gefunden, daß die neuen substituierten Azolylketone und -carbinole der Formel (I) starke fungizide und pflanzenwachstumsregulierende Eigenschaften aufweisen. Dabei zeigen überraschenderweise die erfindungsgemäßen Verbindungen der Formel (I) bessere fungizide und bessere pflanzenwachstumsregulierende Wirkungen als die oben genannten aus dem Stande der Technik bekannten Triazolylalkanone und -ole, welche chemisch ind wirkungsmäßig naheliegende Verbindungen sind. Die erfindungsgemäßen Wirkstoffe stellen somit eine Bereicherung der Technik dar.

Außerdem sind die neuen substituierten Azolylketone und -carbinole der Formel (I) interessante Zwischenprodukte. So können durch entsprechende Umsetzungen funktionelle Derivate der Ketogruppe erhalten werden, wie z.B. Oxime und Oximether, Hydrazone und Ketale. Ferner können die Verbindungen der Formel (I) an der Hydroxygruppe in üblicher Weise in die entsprechenden Ether überführt werden; bzw. können durch Umsetzung mit z.B. Acylhalogeniden oder Carbamoylchloriden in prinzipiell bekannter Weise Acyl- oder Carbamoyl-Derivate der Verbindungen der Formel (I) erhalten werden.

Die erfindungsgemaßen substituierten Azolyl-ketone und -alkohole sind durch die Formel (I) allgemein definiert. In dieser Formel stehen vorzugsweise:

A und B für die in der Erfindungsdefinition angegebenen Bedeutungen;

$R^1$ für geradkettiges oder verzweigtes Alkyl mit 1 bis 6 Kohlenstoffatomen, für geradkettiges oder verzweigtes Alkinyl mit jeweils 2 bis 6 Kohlenstoffatomen, sowie für gegebenenfalls einfach bis zweifach, gleich oder verschieden substituiertes Phenylalkyl mit 1 bis 2 Kohlenstoffatomen im Alkylteil, wobei als Phenylsubstituenten genannt seien: Fluor, Chlor, Methyl, Ethyl, Isopropyl, tert.-Butyl, Methoxy, Methylthio, Cyclohexyl, Dimethylamino, Trifluormethyl, Trifluormethoxy, Trifluormethylthio, Nitro, Cyano, Methoxycarbonyl, oder jeweils gegebenenfalls durch Chlor und Fluor substituierter Phenyl und Phenoxy; ferner für jeweils gegebenenfalls einfach oder zweifach, gleich oder verschieden durch Methyl; Ethyl; Isopropyl oder tert.-Butyl substituiertes Cyclopropyl, Cyclopropylmethyl, Cyclopentyl, Cyclopentylmethyl, Cyclohexyl, Cyclohexylmethyl und Cycloheptyl;

$R^2$ für jeweils einfach oder zweifach, gleich oder verschieden durch Methyl, Ethyl, Isopropyl oder tert.-Butyl substituiertes Cyclopropyl, Cyclopentyl und Cyclohexyl, sowie für die Gruppierung $—(CH_3)_2R^3$, wobei

$R^3$ für geradkettiges oder verzweigtes Alkyl mit 3 bis 6 Kohlenstoffatomen, für Vinyl, Propargyl oder die —CH=O-Gruppe, Methoxyiminomethyl, Dimethoxymethyl, den Dioxolan- und 1,3-Dioxan-Rest steht; und

Bevorzugte erfindungsgemäße Verbindungen sind auch Additionsprodukte aus Säuren und denjenigen substituierten Azolyl-ketonen und -alkoholen der Formel (I), in denen die Substituenten A, B, $R^1$

und $R^2$ die Bedeutungen haben, die bereits vorzugsweise für diese Substituenten genannt wurden.

Zu den Säuren die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Salizylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäß Verbindungen sind Additionsprodukte aus Salzen von Metallen der II. bis IV. Haupt- und der I. und II. sowie IV. bis VIII. Nebengruppe und denjenigen substituierten Azolyl-ketonen und -alkoholen der Formel (I), in denen die Substituenten Ar, B und $R^1$ die Bedeutungen haben, die bereits vorzugsweise für diese Reste gekannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man beispielsweise 3-(Dioxolan-2-yl)-3-methyl-1-(1,2,4-triazol-1-yl)-butan-2-on und 2,4-Dichlorbenzylbromid als Ausgangsstoffe, so kann der Reaktionsablauf durch das folgende Formelschema wiedergegeben werden (Verfahren a):

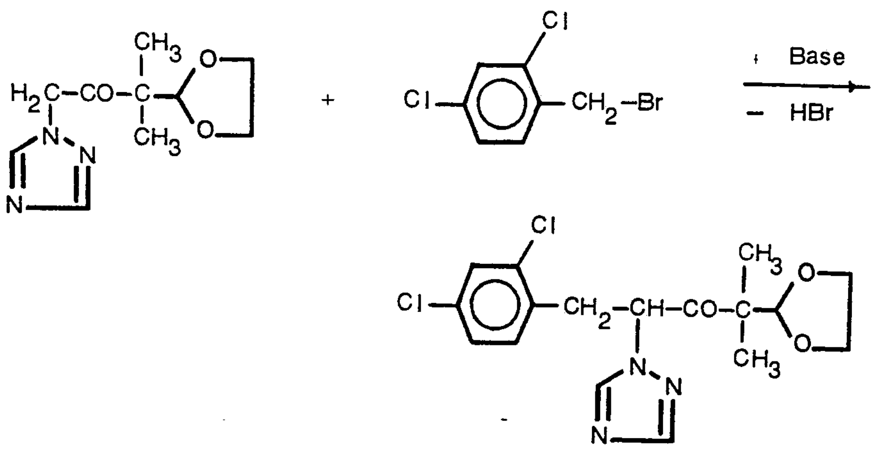


Verwendet man beispielsweise 4-Brom-2-(dioxolan-2-yl)-5-(2,4-dichlorphenyl)-2-methyl-pentan-3-on und 1,2,4-Triazol als Ausgangsstoffe, so kann der Reaktionsablauf durch das folgende Formelschema wiedergegeben werden (Verfahren b):

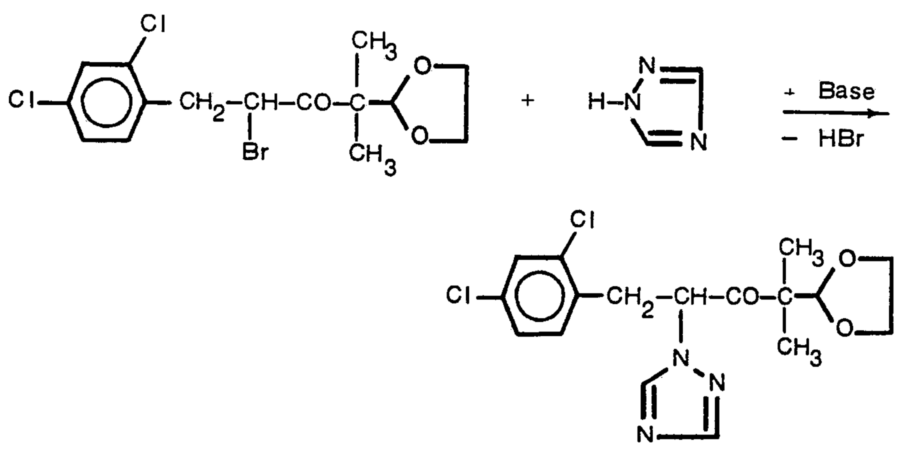


Verwendet man beispielsweise 1-(2,4-Dichlorphenyl)-4-(dioxolan-2-yl)-4-(dioxolan-2-yl)-4-methyl-2-(1,2,4-triazol-1-yl)-pentan-3-on und Natriumborhydrid als Ausgangsstoffe, so kann der Reaktionsablauf durch das folgende Formelschema wiedergegeben werden (Verfahren c):

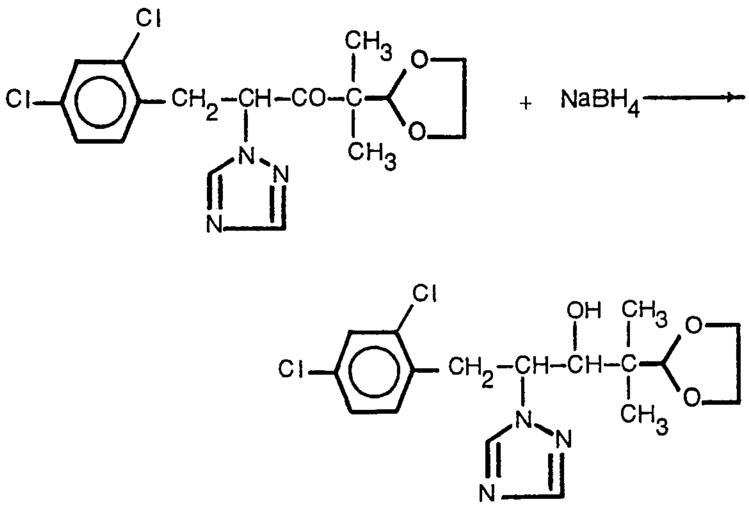


Die bei der Durchführung des erfindungsgemäßen Verfahrens (a) als Ausgangsstoffe zu verwendenden Azolyl-ketone sind durch die Formel (II) allgemein definiert. In dieser Formel stehen A und $R^2$ vorzugsweise für die Reste, die bereits im Zusammenhang ist der Beschreibung der erfindungsgemäßen Stoffe der Formel (I) vorzugsweise für diese Substituenten genannt wurden.

Die Azolyl-ketone der Formel (II) sind teilweise bekannt (vergleiche z.B. DE—A—24 31 407 und DE—A—30 28 330; teilweise sind sie Gegenstand eigener älterer Patentanmeldungen gemäß DE—A—31 45 857, DE—A—31 45 858 und DE—A—32 09 431. Sie werden z.B. durch Umsetzung der entsprechenden Halogenketone mit Imidazol bzw. 1,2,4-Triazol erhalten. Noch nicht bekannt sind Azolyl-ketone der allgemeinen Formel

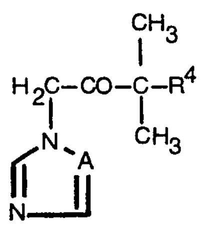

(IIa)

in welcher

A die oben angegebene Bedeutung hat und

$R^4$ für die CHO-Gruppe, Alkoximinomethyl mit 1 bis 4 Kohlenstoffatomen im Alkoxyteil, Dialkoxymethyl mit 1 bis 4 Kohlenstoffatomen in jedem Alkoxyteil sowie für Dioxolanyl und Dioxanyl steht.

Die neuen Azolyl-ketone der Formel (IIa) sind allgemein interessante Zwischenprodukte und können erhalten werden, indem man Halogenmethyl-ketone der Formel

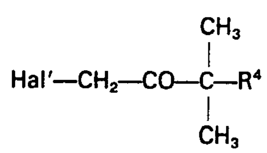

(VI)

in welcher

Hal' für Chlor oder Brom steht und

$R^4$ die oben angegebene Bedeutung hat,

in üblicher Weise mit 1,2,4-Triazol oder Imidazol in Gegenwart eines inerten organischen Lösungsmittels, wie beispielsweise Aceton, und in Gegenwart eines Säurebindemittels, wie beispieslweise Kaliumcarbonat, bei Temperaturen zwischen 20 und 150°C umsetzt.

Die Halogenmethyl-ketone der Formel (VI) sind ebenfalls noch nicht bekannt. Sie werden erhalten, indem man 1-(N-Morpholino)-isobuten der Formel

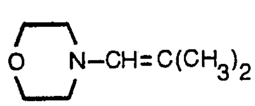

(VII)

mit Halogenacetylchloriden der Formel

$$Hal'—CH_2—CO—Cl \quad (VIII)$$

in welcher

Hal' die oben angegebene Bedeutung hat, in Gegenwart eines Lösungsmittels, wie beispielsweise Diethylether, bei Temperaturen zwischen 20 und 120°C umsetzt und gegebenenfalls die so erholtenen Halogenmethylketone der Formel

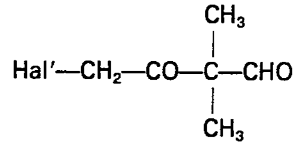

(VIa)

in welcher Hal' die oben angegebene Bedeutung hat, in üblicher Weise an der Aldehydgruppe derivatisiert, wie z.B. durch Umsetzung mit Diolen in Gegenwart eines inerten organischen Lösungsmittels, wie beispielsweise Toluol, und in Gegenwart einer starken Säure als Katalysator, wie beispielsweise p-Toluolsulfonsäure, bei Temperaturen zwischen 80 und 100°C.

Die außerdem für das erfindungsgemäße Verfahren (a) als Ausgangsstoffe zu verwendenden Alkylierungsmittel sind durch die Formel (III) allgemein definiert. In dieser Formel steht $R^1$ vorzugsweise für diejenigen Reste, die bereits im Zusammenhang mit der Beschreibung der erfindungsgemäßen Stoffe der Formel (I) vorzugsweise für diesen Substituenten genannt wurden. Z steht vorzugsweise für eine elektronenanziehende Abgangsgruppierung, wie beispielsweise Halogen, p-Methylphenylsulfonyloxy, die Gruppierung $—O—SO_2—OR$ oder $—NR_3$ und andere (R steht hierbei z.B. für Alkyl mit 1 bis 4 Kohlenstoffatomen).

Die Alkylierungsmittel der Formel (III) sind allgemein bekannte Verbindungen.

Die bei der Durchführung des erfindungsgemäßen Verfahrens (b) als Ausgangsstoffe zu verwendenden Halogenketone sind durch die Formel (IV) allgemein definiert. In dieser Formel stehen $R^1$ und $R^2$ vorzugsweise für die Reste, die bereits im Zusammenhang mit der Beschreibung der erfindungsgemäßen Stoffe der Formel (I) vorzugsweise für diese Substituenten genannt wurden.

Die Halogenketone der Formel (IV) sind noch nicht bekannt; sie können jedoch in allgemein bekannter Art und Weise erhalten werden, indem man z.B. die entsprechenden Ketone mit Chlor oder Brom in Gegenwart eines inerten organischen Lösungsmittels, wie beispielsweise chlorierten Kohlenwasserstoffen, bei Raumtemperatur umsetzt, oder mit üblichen Chlorierungsmitteln, wie beispielsweise Sulfurylchlorid, bei Temperaturen zwischen 20 und 60°C umsetzt.

Die außerdem für das erfindungsgemäße Verfahren (b) als Ausgangsstoffe zu verwendenden Azole sind durch die Formel (V) allgemein definiert. In dieser Formel steht A vorzugsweise für die in der Erfindungsdefinition angegebenen Bedeutungen.

Die Azole der Formel (V) sind allgemein bekannte Verbindungen.

Die bei der Durchführung des erfindungsgemäßen Verfahrens (c) als Ausgangsstoffe zu verwendenden Verbindungen sind durch die Formel (Ia) allgemein definiert. Die Verbindungen der Formel (Ia) sind erfindungsgemäße Stoffe.

Für das erfindungsgemäße Verfahren (a) kommen als Verdünnungsmittel inerte organische Lösungsmittel infrage. Hierzu gehören vorzugsweise aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; Ester, wie Essigester; Formamide, wie Dimethylformamid; sowie Dimethylsulfoxid.

Das erfindungsgemäße Verfahren (a) wird in Gegenwart einer Base durchgeführt. Hierbei können alle üblichen organischen und insbesondere anorganischen Basen eingesetzt werden, wie vorzugweise alkalihydroxide oder Alkalicarbonate, beispielsweise seien Natrium- und Kaliumhydroxid genannt.

Die Reaktionstemperaturen können bei der Durchführung des erfindungsgemäßen Verfahrens (a) in einem größeren Bereich variiert werden. Im allgemeinen arbeitet man zwischen 0 und 100°C, vorzugsweise zwischen 20 und 100°C.

Bei der Druchführung des erfindungsgemäßen Verfahrens (a) arbeitet man vorzugsweise in äquimolaren Mengen. Die Isolierung der Endprodukte der Formel (I) erfolgt in allgemein üblicher Art und Weise.

Das erfindungsgemäße Verfahren (a) kann auch in einem Zweiphasensystem wie beispielsweise wässrige Natron- oder Kalilauge/Toluol oder Methylenchlorid, gegebenenfalls unter Zusatz von 0,1 bis 1 Mol eines Phasentransferkatalysators, wie beispielsweise Ammonium-oder Phosphoniumverbindungen, beispielsweise seien Benzyldodecyl-dimethylammoniumchlorid und Triethyl-benzyl-ammoniumchlorid genannt, durchgeführt werden.

Für das erfindungsgemäße Verfahren (b) kommen als Verdünnungsmittel inerte organische Lösungsmittel infrage. Hierzu gehören vorzugsweise Ketone, wie Diethylketon und insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; aromatische Kohlenwasserstoffe, wie Toluol, Benzol oder Chlorbenzol; Formamide, wie insbesondere Dimethylformamid; und halogenierte Kohlenwasserstoffe.

Das erfindungsgemäße Verfahren (b) wird in Gegenwart eines Säurebindemittels vorgenommen. Man kann alle üblicherweise verwendbaren anorganischen und organischen Säurebinder zugeben, wie Alkalicarbonate, beispielsweise Natriumcarbonat, Kaliumcarbonat und Natriumhydrogen-carbonat, oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan.

Vorzugsweise verwendet man einen entsprechenden Ueberschuß an Azol.

Die Reaktionstemperaturen können bei dem erfindungsgemäßen Verfahren (b) in einem größeren Bereich variiert werden. Im allgemeinen arbeitet man zwischen etwa 20 bis etwa 150°C, vorzugsweise bei 40 bis 100°C. Bei anwesenheit eines Lösungsmittels wird zweckmäßigerweise beim Siedepunkt des jeweiligen Lösungsmittels gearbeitet.

Bei der Durchführung des erfindungsgemäßen Verfahrens (b) setzt man auf 1 Mol der Verbindung der Formel (IV) vorzugsweise 2 bis 4 Mol Azol und 1 bis 4 Mol Säurebinder ein. Zur Isolierung der Verbindungen der Formel (I) wird das Lösungsmittel abdestilliert und der Rückstand in üblicher Art und Weise aufgearbeitet.

Die erfindungsgemäße Reduktion gemäß Verfahren (c) erfolgt in üblicher Weise, z.B. durch Umsetzung mit komplexen Hydrigen, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Butanol, Isopropanol und Ether, wie Diethylether oder Tetrahydrofuran. Die Reaktion wird im allgemeinen bei 0 bis 30°C, vorzugsweise bei 0 bis 20°C durchgeführt. Hierzu setzt man auf 1 Mol des Ketons der Formel (I) etwa 1 Mol eines komplexen Hydrids, wie Natriumborhydrid oder Lithiumalanat ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des Ketons der Formel (I) etwa 0,3 bis 2 Mol Aluminiumisopropylat ein. Zur Isolierung der reduzierten Verbindungen der Formel (I) wird das überschüssige Lösungsmittel in Vakuum entfernt und die entstandenen Aluminium-Verbindungen mit verdünnter Schwefelsäure oder Natronlauge zersetzt. Die weitere Aufarbeitung erfolgt in üblicher Weise.

Die erfindungsgemäß verwendbaren Wirkstoffe greifen in den Metabolismus der Pflanzen ein un können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß des Getreide lagert.

Eine Hemmung des vegetativen Wachstums ermöglicht bei vielen Kulturpflanzen eine dichtere Anpflanzung, sodaß Mehrerträge bezogen auf die Sodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderng des vegetativen Wachstums erzielen. Dies ist von großem Nutzen, wenn die vegetativen Pflanzenteile geerntet werden. Eine Forderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann des Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikalcominanz die Entwicklung von Seitentrieben gefördert weden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zumvermeiden.

Schließlich kann mit Wachstumsregulatoren eine Resistenz der Pflanzen gegen Frost, Trockenheit oder hohen Salzegehalt des Bodens induziert werden. Hierdurch wird die Kultivierung von Pflanzen in Gebieten möglich, die hierzu normalerweise ungeeignet sind.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäß verwendbaren Wirkstoffe mit besonders gutem Erfolgt zur Bekämpfung solcher Pilze eingesetzt werden, die echte Mehltauerkrankungen hervorrufen; so zur Bekämpfung von Erysiphe-Arten, wie z.B. gegen den Erreger des Gersten- bzw. Getreidemehltaus (Erysiphe graminis) oder von Sphaerotheca-Arten, wie z.B. gegen den Erreger des Gurkenmehltaus (Sphaerotheca fuligenea); ferner zur Bekämpfung von weiteren Getreidekrankheiten, wie Cochliobolus sativus und Pyrenophora teres, sowie von Reiskrankheiten, wie Pyricularia oryzae und Pellicularia sasakii. Hervorzuheben ist die zusätzliche bakterizide Wirkung.

Die Wirkstoffe können in die üblichen Formulierungen übergeführt werden, wie Lösungen,

Emulsionen, Suspension, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch vermischen der Wirkstoffemit Streckmitteln, also flüssigen Lösungsmitteln, unter Druck stehenden verflüssigten Gasen und/oder esten Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B gebrochene un fraktionierte natürliche Gestein wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeihen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Auch beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugweise zwischen 0,5 und 0,001%. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02% am Wirkungsort erforderlich.

Herstellungsbeispiele
Beispiel 1

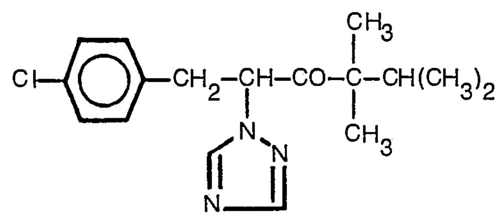

(Verfahren a)

15,6 g (0,08 Mol) 1-(1,2,4-Triazol-1-yl)-3,3,4-trimethylpentan-2-on werden in 100 ml Dimethylsulfoxid gelöst und 4,5 g (0,08 Mol) Kaliumhydroxid, gelöst in 10 ml Wasser, unter Kühlung zugegeben. Dazu werden 16,4 g (0,08 Mol) 4-Chlorbenzylbromid, gelöst in 50 ml Dimethylsulfoxid, so zugetropft, daß die Temperatur nicht über 40°C steigt. Danach wird langsam auf 100°C erwärmt, 15 Stunden bei dieser Temperatur nachgerührt und die abgekühlte Lösung auf 500 ml Wasser gegossen. Man extrahiert zweimal mit je 250 ml Methylenchlorid, danach die Methylenchloridphase viermal mit je 100 ml Wasser und engt die organische Phase ein. Der Rückstand wird in 100 ml Aceton aufgenommen und mit 14,4 g 1,5-Naphthalindisulfonsäure, gelöst in 50 ml Aceton, versetzt. Es kristallisiert das 1,5-Naphthalindisulfonsäure-Salz von 2,3,3-Trimethyl-5-(1,2,4-triazol-1-yl)-6-(4-chlorphenyl)-hexan-3-on aus. Der Niederschlag wird abgesaugt, in 250 ml gesättigter Natriumhydrogencarbonatlösung aufgenommen, die wässrige Phase viermal mit je 100 ml Methylenchlorid extrahiert, die Methylenchloridphase mit 100 ml Wasser exrahiert und von der organischen Phase das Lösungsmittel abdestilliert. Man erhält 17,3 g (67,6% der Theorie) 2,3,3-Trimethyl-5-1,2,4-triazol-1-yl)-6-(4-chlorphenyl)-hexan-4-on als farblose Kristalle vom Schmelzpunkt 91—94°C.

Herstellung des Ausgangsproduktes

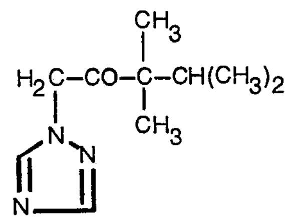

162,5 g (1 Mol) 1-Chlor-3,3,4-trimethyl-pentan-2-on werden zu einer Suspensions von 84 g (1,2 Mol) 1,2,4-Triazol, 165,6 g (1,2 Mol) Kaliumarbonat in 1 l Ethanol zugetropft. Man rührt 15 Stunden bei 40°C, saugt den anorganischen Niederschlag ab, destilliert das Lösungsmittel ab, nimmt den Niederschlag in 500 ml Methylenchlorid auf, extrahiert die organische Phase mit 1 l Wasser, extrahiert die wässrige Phase mit 500 ml Methylenchlorid, vereinigt die Methylenchloridphasen und extrahiert zweimal mit je 1 l Wasser. Von der organischen Phase wird das Lösungsmittel abdestilliert. Man erhält 154,6 g (79,3% der Theorie) 1-(1,2,4-Triazol-1-yl)-3,3,4-trimethyl-pentan-2-on als farbloses Oel vom Brechungsindex $n_D^{20}$ = 1,4827.

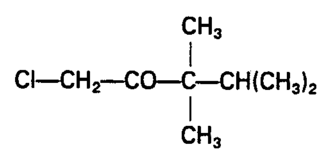

238 g (1 Mol) 1-Chlor-2-phenoxy-3,3,4-trimethyl-1-penten werden mit 500 g Ameisensäure und 50 g konzentrierter Salzsäure 2 Stunden bie 80°C gerührt. Man verdünnt mit Methylenchlorid, wäscht einmal mit Wasser und viermal mit verdünnter natronlauge. Nach dem Trocknen über Natriumsulfat wird das Lösungsmittel im Vakuum abgezogen und der Rückstand über eine Kolonne destilliert. Man erhält 125 bis 135 g (77—83% der Theorie) 1-Chlor-3,3,4-trimethyl-2-pentanon von Siedepunkt 88—93°C/16 mbar.

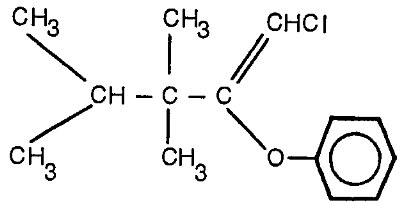

In 1,6 l ca. 100°C heißes N-Methylpyrrolidon werden 487 g Natriumphenolat eingetragen und durch Erhitzen auf 200°C gelöst. Im Verlauf von 3 Stunden werden 360 g (2 Mol) 1,1-Dichlor-3,3,4-trimethyl-1-penten so zugetropft, daß die Temperatur der Reaktionsmischung nicht unter 195°C sinkt. Anschließend erhitzt man 8 Stunden auf 200—210°C. Zur Aufarbeitung wird die Lösung mit Methylenchlorid verdünnt und mit verdünnter Natronlauge ausgeschüttelt. Nach Trocknen und Abziehen des Lösungsmittels bleiben 406 g Rohprodukt zurück, das bei 105—120°C/0,1 mbar destilliert wird. Man erhält 337 g (71% der Theorie) 1-Chlor-2-phenoxy-3,3,4-trimethyl-1-penten.

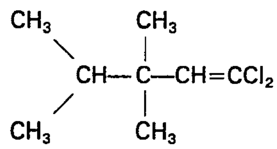

In einer trockenen Apparatur mit Trockenrohr gibt man 20 g wasserfreies, gepulvertes Aluminiumchlorid portionsweise zu 2040 g (21 Mol) Vinylidenchlorid bei −10°C. Dann tropft man im Temperaturbereich von 0 bis 10°C 837 g (7 Mol) 2-Chlor-2,3-dimethylbutan zu. Man läßt auf 20°C erwärmen und gibt weitere Aluminiumchlorid-Portionen (max. 20 g) unter Kühlung zu, bis die Reaktion nicht mehr exotherm ist. Der Ansatz wird 4 Stunden bei Raumtemperatur nachgerührt, durch Eintropfen von 70 ml Essigsäure wird der Katalysator desaktiviert und die Mischung wird über Natriumsulfat filtriert. Durch Eintropfdestillation bei 1 mbar werden die flüchtigen Bestandteile von den schwerflüchtigen getrennt.

Das Destillat wird an einer Kolonne fraktioniert. Man erhält 824 bis 1226 g (65—95% der Theorie) 1,1-Dichlor-3,3,4-trimethyl-1-penten vom Siedepunkt 60—75°C/20 mbar.

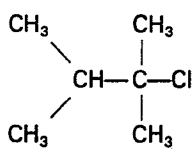

In 840 g (10 Mol) 2,3-Dimethyl-1-buten werden bei 10 bis 20°C unter Eiskühlung ca. 440 g (12 Mol) Chlorwasserstoff aus der Bombe in 16 Stunden eingeleitet. Laut IR ist vollständiger Umsatz erfolgt. Ueberschüssiger Chlorwasserstoff wird in die Waserstrahlpumpe gezogen. Man erhält 1103 g (91% der Theorie) 2-Chlor-2,3-dimethyl-butan.

Beispiel 2

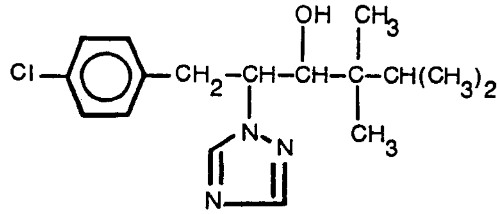

(Verfahren c)

133,8 g (0,043 Mol) 2,3,3-Trimethyl-5-(1,2,4-triazol-1-yl)-6-(4-chlorphenyl)-hexan-4-on (Beispiel 1) werden in 100 ml Methol gelöst und bei 0 bis 10°C werden portionsweise 2,1 g (0,056 Mol) Natriumborhydrid zugegeben, sodann läßt man 15 Stunden bei Raumtemperatur nachreagieren, tropft 90 ml 2n Salzsäure zu und läßt weitere 15 Stunden bei Raumtemperatur nachrühren.

Man gibt 300 ml gesättigte Natriumhydrogencarbonatlösung zu, extrahiert zweimal mit je 250 ml Methylenchlorid, wäscht mit je 250 ml Methylenchlorid, wäscht die Methylenchloridphase dreimal mit je 50 ml Wasser und destilliert das Methylenchlorid ab. Man erhalt 10 g (72,5 g der Theorie) 2,3,3-Trimethyl-5-(1,2,4-triazol-1-yl)-6-(4-chlorphenyl)hexan-4-ol als farblose kristalle vom Schmelzpunkt 106—108°C.

Beispiel 3

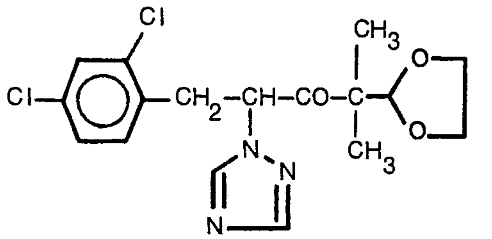

x 1/2

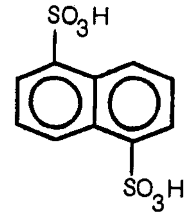

(Verfahren a)

34 g (0,15 Mol) 1-(1,2,4-Triazol-1-yl)-3-(dioxolan-2-yl)-3-methyl-butan-2-on werden in 150 ml Dimethylsulfoxid vorgelegt, 8,4 g gepulvertes Kalimhyroxid bei 15°C zugegeben und 36 g (0,15 Mol) 2,3-Dichlorbenzylbromid, in 30 ml Dimethylsulfoxid gelöst, zugetropft. Das Reaktionsgemisch wird bei 20°C 15 Stunden nachgerührt und die Suspension auf 500 ml Wasser gegossen. Man extrahiert mit 600 ml Methylenchlorid, wäscht die organische Phase zweimal mit je 1 l Wasser und destilliert das Lösungsmittel ab. Der ölige Rückstand wird in 600 ml Aceton gelöst und mit 1,5-Naphthalindisulfonsäure in 60 ml Aceton versetzt. Bei 0°C erfolgt nach 6 Stunden Kristallisation. Man erhält 50 g (38,6% der Theorie) 1-(2,4-Dichlorphenyl)-2-(1,2,4-triazol-1-yl)-4-(dioxolan-2-yl)-4-methyl-pentan-3-on -1,5-naphthalindisulfonat vom Schmelzpunkt 199—210°C.

Herstellung des Ausgangsproduktes

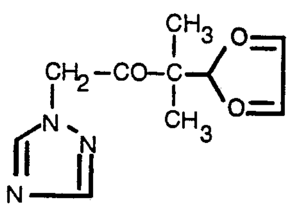

180,6 g (0,94 Mol) 1-Chlor-3-(dioxolan-2-yl)-3-methylbutan-2-on, 64,9 g (0,94 Mol) 1,2,4-Triazol und 142,7 g (1 Mol) Kaliumcarbonat werden in 1000 ml Methylethylketon 16 Stunden unter Rückfluß erhitzt. Der Feststoff wird abgesaugt und das Lösungsmittel abdestilliert, der ölige Rückstand in 700 ml Methylenchlorid aufgenommen und die organische Phase zweimal mit je 1000 ml Wasser extrahiert. Von der organischen Phase wird das Lösungsmittel abdestilliert. Man erhält 151,3 g (71,5% der Theorie) 1 - (1,2,4 - Triazol - 1 - yl) - 3 - (dioxolan - 2 - yl) - 3 - methyl - butan - 2 - on als Oel mit gaschromatographischer Reinheit von 99%.

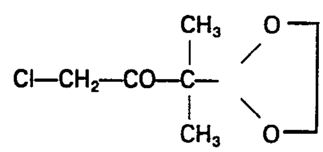

204 g (1,38 Mol) 2,2-Dimethyl-4-chlor-3-keto-butanol werden mit 93 g (1,5 Mol) Ethylenglykol und 0,7 g p-Toluolsulfonsäure in 400 ml Methylenchlorid 3 Stunden am Wasserabscheider erhitzt. Die organische Phase wird mit 150 ml 5%-iger Natronlauge und danach mit 400 ml Wasser extrahiert. Das Lösungsmittel wird abdestilliert und der Rückstand im Wasserstrahlvakuum destilliert. Man erhält 211 g (79,8% der Theorie) 1-Chlor-3-(dioxolan-2-yl)-3-methyl-butan-2-on vom Siedepunkt 127—28°C/14 mbar.

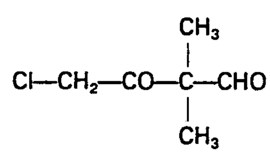

210 g (1,5 Mol) 1-morpholino-2-methyl-1-propan werden innerhalb einer Stunde zu 169,0 g (1,5 Mol) Chloracetylchlorid, gelöst in 350 ml Diethylether, bei 5°C zugetropft. Nach beendeter Zugabe rührt man weitere 3 Studen unter Rückfluß. Die Lösung wird auf 100 g Eis gegossen,mit wässriger Natriumhydrogencarbonatlösung auf pH 5 gebracht und die Etherphase abgetrennt. Die Wasserphase wird mit 100 ml Diethylether extrahiert, die organischen Phasen vereinigt, über Natriumsulfat getrocknet, das Lösungsmittel abdestilliert und der Rückstand im wasserstrahlvakuum destilliert.

Man erhält 136,4 g (61% der Theorie) 4-Chlor-3-keto-2,2-dimethyl-butanal vom Siedepunkt 95—98°C/14 mbar.

Beispiele 4 und 5

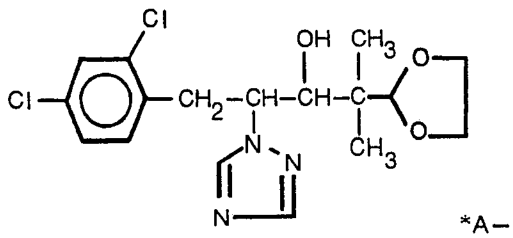

Beispiel 4: B–Form*

Beispiel 5: A–Form*

*A– und B–Form: die beiden möglichen geometrischen Isomeren

(Verfahren c)

22,9 g (0,06 Mol) 1 - (2,4 - Dichlorphenyl) - 2 - (1,2,4 - triazol - 1 - yl) - 4 - (dioxolan - 2 - yl) - 4 - methyl - pentan - 3 - on werden in 100 ml Methanol gelöst, auf 0°C abgekühlt und portionsweise 2,95 g (0,078 Mol) Natriumborhydrid zugegeben. Man läßt 3 Stunden bei Raumtemperatur nachrühren, 15 ml konzentrierte Salzsäure zutropfen, weitere 3 Stunden nachrühren und gibt dann 500 ml gesättigte Natriumhydrogencarbonatlösung zu. Man extrahiert mit 400 ml Methylenchlorid, trennt die Phasen, wäscht die organische Phase zweimal mit je 100 ml Wasser und destilliert das Lösungsmittel ab. Der Rückstand wird in 150 ml Diisopropylether aufgenommen und die Kristalle abgesaugt. Man erhält 1 - (2,4 - Dichlorphenyl) - 2 - (1,2,4 - triazol - 1 - yl) - 4 - (dioxolan - 2 - yl) - 4 - methyl - pentan - 3 - ol als B-Form, vom Schmelzpunkt 178—81°C und nach Abdestillation von ca. 100 ml Diisopropylether 8,9 g als A-Form vom Schmelzpunkt 95—100°C.

Beispiel 6

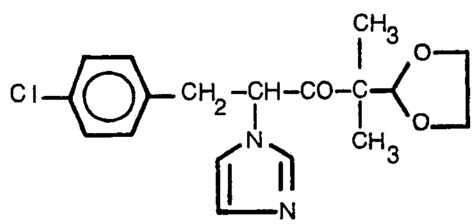

(Verfahren a)

30 g (0,131 Mol) 1-(Imidazol-1-yl)-3-(dioxolan-2-yl)-3-methyl-butan-2-on werden in 130 ml Dimethylsulfoxid gelöst, bei 10°C 7,5 g gepulvertes Kaliumhydroxid zugegeben und 21 g 4-Chlorbenzylchlorid (0,131 Mol), gelöst in 30 ml Dimethylsulfoxid, zugetropft. Nach 15 Stunden Rühren bei 20°C wird die Suspension auf 500 ml Wasser gegossen, mit 600 ml Methylenchlorid extrahiert, die organische Phase wird mit je 1 l Wasser extrahiert, das Lösungsmittel abdestilliert. Der ölige Rückstand wird in 300 ml Diethylether aufgenommen, die Etherphase filtriert und das Lösungsmittel abdestilliert. Man erhält 14 g (30,6% der Theorie) 1 - (4 - Chlorphenyl) - 4 - (dioxolan - 2 - yl) - 2 - (imidazol - 1 - yl) - 4 - methyl - pentan - 3 - on vom Brechungsindex $n_D^{20} = 1{,}5490$.

Herstellung des Ausgangsproduktes

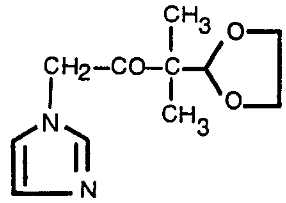

106,8 g (0,55 Mol) 1-Chlor-3-(dioxolan-2-yl)-3-methylbutan-2-on und 74,8 g (1,1 Mol) Imidazol werden in 500 ml Acetonitril 15 Stunden bei 65°C gerührt. Das Lösungsmittel wird abdestilliert, der Rückstand in 800 ml Methylenchlorid aufgenommen und zweimal mit je 1 l Wasser extrahiert. Die Methylenchloridphase Wird über Natriumsulfat getrocknet und das Lösungsmittel abdestilliert.

Man erhält 71,7 g (57,7% der Theorie) 1-(Imidazol-1-yl)-3-(dioxolan-2-yl)-3-methyl-butan-2-on (gaschromatographischer Gehalt 95,4%).

Beispiel 7

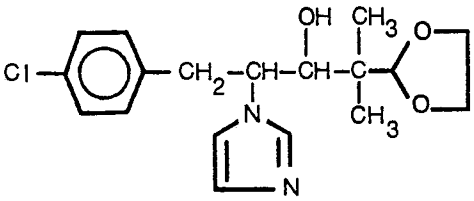

(Verfahren c)

14,6 g (0,042 Mol) 4 - (Dioxolan - 2 - yl) - 4 - methyl - 1 - (4 - chlorphenyl) - 2 - (imidazol - 1 - yl) - pentan - 3 - on werden in 150 ml Methanol gelöst und 2,065 g (0,055 Mol) Natrimborhydrid bei 0°C portionsweise zugegeben; nach 3 Stunden Reaktionszeit bei Raumtemperatur werden 15 ml Salzsäure (konzentriert) zugetropft und weitere 3 Stunden bei Raumtemperatur nachgerührt, sodann wird mit 500 ml gesättigter wäßriger Natriumhydrogencarbonatlösung versetzt. Man extrahiert mit 400 ml Methylenchlorid, trennt die Phasen, wäscht die organische Phase zweimal mit 100 ml Wasser und destilliert das Lösungsmittel ab. Der Rückstand wird in 150 ml Diisopropylether aufgenommen und auf 0°C abgekühlt. Dabei kristallisieren 10,8 g (73,8% der Theorie)4 - (Dioxolan - 2 - yl) - 4 - methyl - 1 - (4 - chlorphenyl) - 2 - (imidazol - 1 - yl) - pentan - 3 - ol vom Schmelzpunkt 151—157°C aus.

In analoger Weise und entsprechend den angegebenen Verfahrensvarianten werden die folgenden Verbindungen der allgemeinen Formel

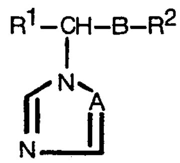

(I)

erhalten:

| Bsp. Nr. | $R^1$ | $R^2$ | A | B | Schmelzpunkt (°C) bzw. $n_D^{20}$ |
|---|---|---|---|---|---|
| 8 | 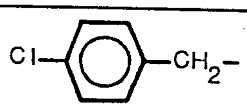 | $—C(CH_3)_2—CH=CH_2$ | N | CO | 43—48 |
| 9 | 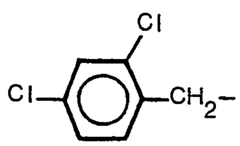 | $—C(CH_3)_2—C_3H_7—i$ | N | CO | 98—102 |
| 10 | 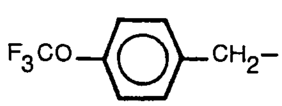 | $—C(CH_3)_2—C_3H_7—i$ | N | CO | 1,485 |
| 11 | 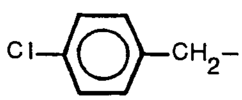 | 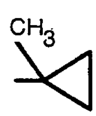 | N | CO | 60 |
| 12 | 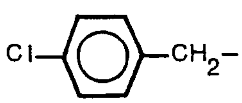 | $—C(CH_3)_2—CH=CH_2$ | N | CH(OH) | 132—46 |
| 13 | 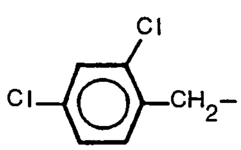 | $—C(CH_3)_2—C_3H_7$-i | N | CH(OH) | 104—11 |
| 14 | 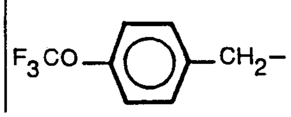 | $—C(CH_3)_2—C_3H_7$-i | N | CH(OH) | 1,489 |

| Bsp. Nr. | $R^1$ | $R^2$ | A | B | Schmelzpunkt (°C) bzw. $n_D^{20}$ |
|---|---|---|---|---|---|
| 15 | 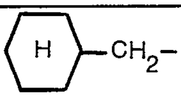 | $—C(CH_3)_{32}—C_3H_7$-i | N | CH(OH) | 1,495 |
| 16 | n-$C_4H_9$— | $—C(CH_3)_2—C_3H_7$-i | N | CH(OH | 1,485 |
| 17 | 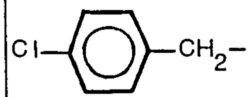 | 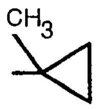 | N | CH(OH) | 118—20 (x HCl) |
| 18 | 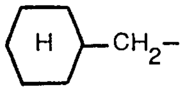 | 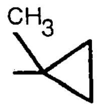 | N | CH(OH) | 160—66 (x HCl) |
| 19 | 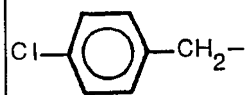 | 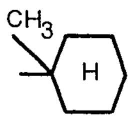 | N | CH(OH) | 170—72 (x HCl) |
| 20 | n-$C_4H_9$— | 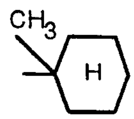 | N | CH(OH) | 156—60 (x HCl) |
| 21 | 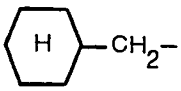 | 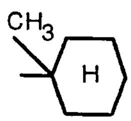 | N | CH(OH) | 125—27 (x HCl) |
| 22 | 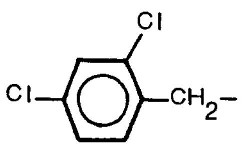 | 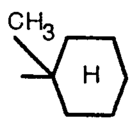 | N | CH(OH) | 165 (x HCl) |
| 23 | 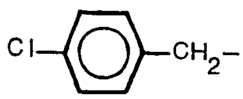 | 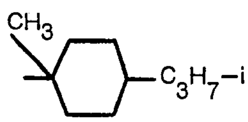 | N | CH(OH) | 130 |
| 24 | n-$C_4H_9$ | 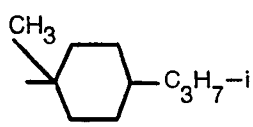 | N | CH(OH) | 94 (Zers.) |
| 25 | 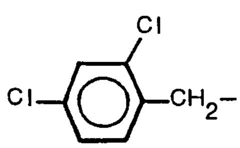 | 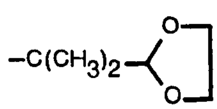 | CH | CO | 69—74 |
| 26 | 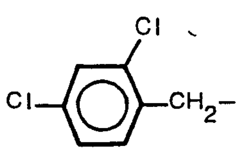 | 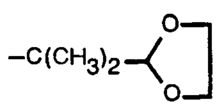 | CH | CH(OH) | 160—67 |
| 27 | 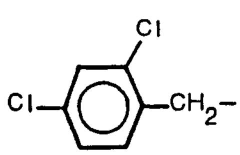 | 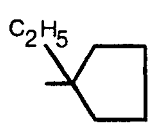 | N | CH(OH) | 138—45 (x HCl) |
| 28 | 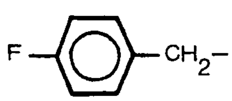 | $—C(CH_3)_2—C_3H_7$-i | N | CH(OH) | 127—30 (x HCl) |

| Bsp. Nr. | $R^1$ | $R^2$ | A | B | Schmelzpunkt (°C) bzw. $n_D^{20}$ |
|---|---|---|---|---|---|
| 29 | 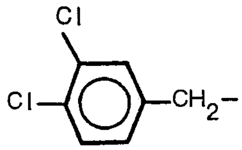 | $—C(CH_3)_2—C_3H_7$-i | N | CH(OH) | 166—67 (x HCl) |
| 30 | n-$C_4H_9$ | $—C(CH_3)_2—CH_2—C(CH)_3$ | N | CH(OH) | 128—32 (x HCl) |
| 31 | n-$C_4H_9$ | $—C(CH_3)_2—CH{=}CH_2$ | N | CH(OH) | 137—38 (x HCl) |
| 32 | 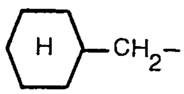 | $—C(CH_3)_2—CH{=}CH_2$ | N | CH(OH) | 156—58 (x HCl) |
| 33 | 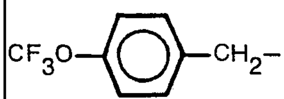 | $—C(CH_3)_2—CH{=}CH_2$ | N | CH(OH) | 148—52 (x HCl) |
| 34 | 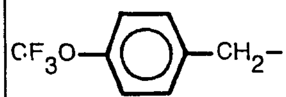 | $—C(CH_3)_2—CH{=}CH_2$ | N | CH(OH) | 168—72 (x HCl) |
| 35 | 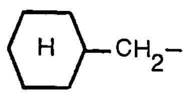 | $—C(CH_3)_2—C_3H_7$-n | N | CO | 1,4881 |
| 36 | $(CH_3)_2CHCH_2CH_2—$ | $—C(CH_3)_2—C_3H_7$-n | N | CO | zähes Öl |
| 37 | n-$C_4H_9$ | $—C(CH_3)_2—C_3H_7$-n | N | CO | 1,4748 |
| 38 | n-$C_4H_9$ | $—C(CH_3)_2—C_3H_7$-n | N | CH(OH) | 1,4809 |
| 39 | 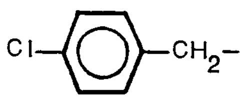 | $—C(CH_3)_2—C_3H_7$-n | N | CO | 83 |
| 40 | 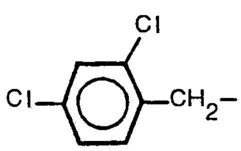 | $—C(CH_3)_2—C_3H_7$-n | N | CO | 76 |
| 41 | 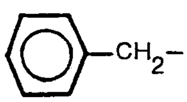 | $—C(CH_3)_2—C_3H_7$-n | N | CO | 1,5209 |
| 42 | $(CH_3)_2CHCH_2CH_2$ | $—C(CH_3)_2—C_3H_7$-n | N | CH(OH) | 1,4789 |
| 43 | 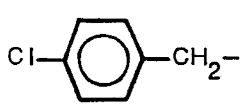 | $—C(CH_3)_2—C_3H_7$-n | N | CH(OH) | 106 |
| 44 | 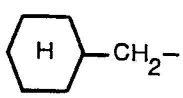 | $—C(CH_3)_2—C_3H_7$-n | N | CH(OH) | 1,4941 |
| 45 | 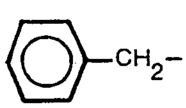 | $—C(CH_3)_2—C_3H_7$-n | N | CH(OH) | 1,5240 |

| Bsp. Nr. | $R^1$ | $R^2$ | A | B | Schmelzpunkt (°C) bzw. $n_D^{20}$ |
|---|---|---|---|---|---|
| 46 | 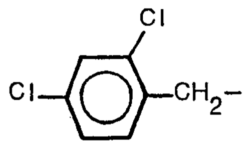 | $-C(CH_3)_2-C_3H_7$-n | N | CH(OH) | 90—92 |
| 47 | n-$C_6H_{13}$ | $-C(CH_3)_2-C_3H_7$-i | N | CH(OH) | 150 (x HCl) |
| 48 | 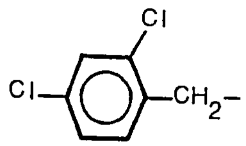 | $-C(CH_3)_2-CH_2-C(CH_3)_3$ | N | CH(OH) | 105—08 |
| 49 | 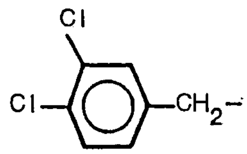 | 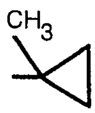 | N | CH(OH) | 168—74 (x HCl) |
| 50 | 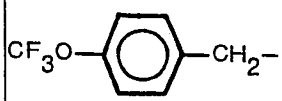 | 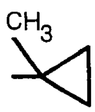 | N | CH(OH) | 148—50 (x HCl) |
| 51 | 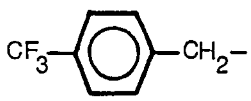 | 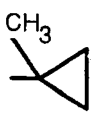 | N | CH(OH) | 182—95 (x HCl) |

| Bsp. Nr. | $R^1$ | $R^2$ | A | B | Fp (°C) $n_D^{20}$ |
|---|---|---|---|---|---|
| 52 | 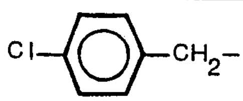 | $-C(CH_3)_2-CH(OCH_3)_2$ | N | CO | 1,5275 |
| 53 | 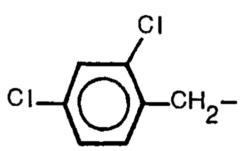 | $-C(CH_3)_3-CH(OCH_3)_2$ | N | CO | 85—87 |
| 54 | 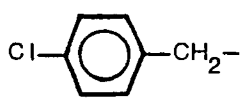 | $-C(CH_3)_2-CH(OCH_3)_2N$ | N | CH(OH) | 1,5330 |
| 55 | 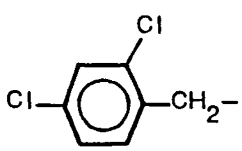 | $-C(CH_3)_2-CH(OCH_3)_2$ | N | CH(OH) | 106—110 |
| 56 | 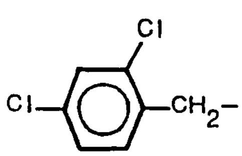 | $-C(CH_3)_2-CHO$ | N | CH(OH) | 141—44 |
| 57 | 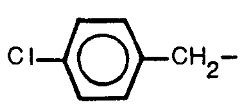 | $-C(CH_3)_2-CH(OCH_3)_2$ | CH | CH(OH) | 154—60 |

| Bsp. Nr. | $R^1$ | $R^2$ | A | B | Fp (°C) $n_D^{20}$ |
|---|---|---|---|---|---|
| 58 | 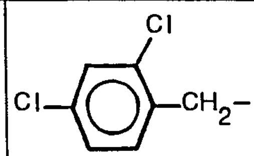 | $—C(CH_3)_2—CH(OCH_3)_2$ | CH | CH(OH) | 132—34 |
| 59 | 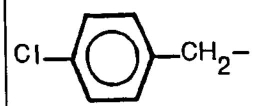 | $—C(CH_3)_2—CH{=}NOCH_3$ | N | CH(OH) | 175—77 (x HCl) |
| 60 | 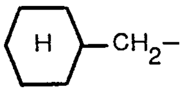 | 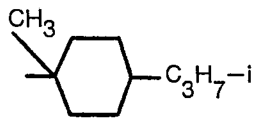 | N | CH(OH) | 74—76 |
| 61 | $n\text{-}C_4H_9\text{-}$ | 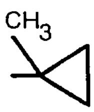 | N | CH(OH) | 72—75 |
| 62 | 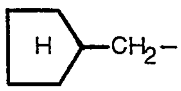 | $—C(CH_3)_2—CH{=}CH_2$ | N | CH(OH) | 144—48 (x HCl) |
| 63 | 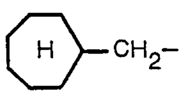 | $—C(CH_3)_2—CH{=}CH_2$ | N | CH(OH) | 131—66 (x HCl) |
| 64 | 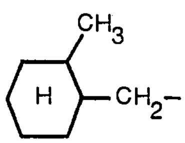 | $—C(CH_3)_2—CH{=}CH_2$ | N | CH(OH) | 114—96 (x HCl) |

Verwendungsbeispiele

In den nachfolgenden Beispielen werden die nachstehend angegebenen Verbindungen als Vergleichssubstanzen eingesetzt:

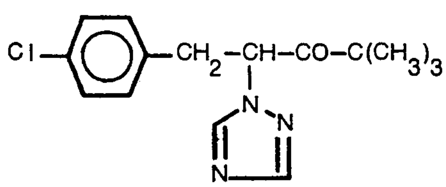

(A)

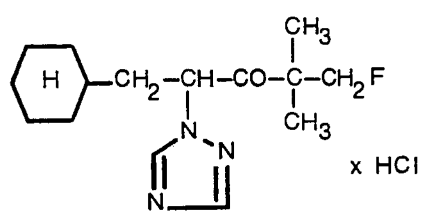

x HCl

(B)

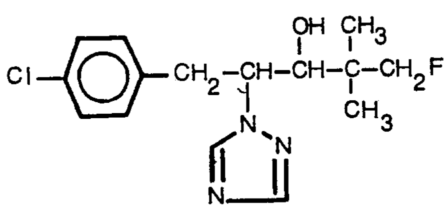

(C)

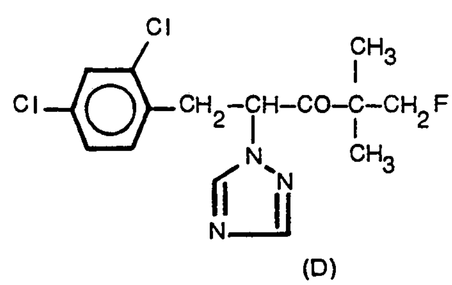

(D)

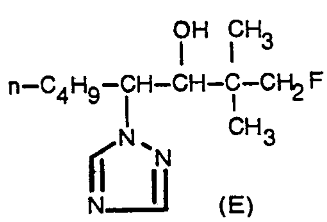

(E)

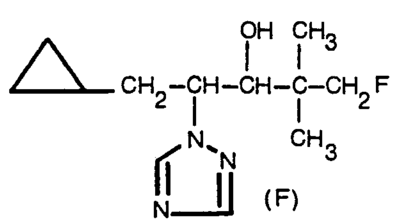

(F)

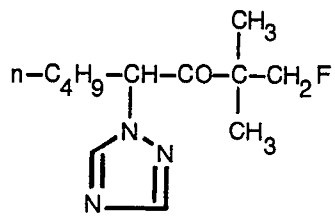

(G)

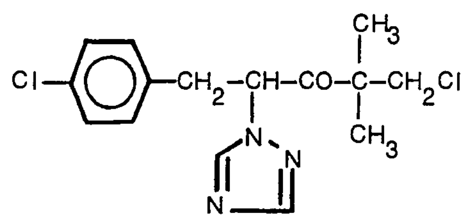

(H)

Beispiel A

Sphaerotheca-Test (Gurke)/protektiv

Lösungsmittel: 4,7 Gewichtsteile Aceton

Emulgator: 0,3 Gewichtsteile Alkylalrylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fulingiea bestäubt.

Die Pflanzen werden anschließend bei 23 bis 24°C und bei einer relativen Luftfeuchtigkeit von ca. 75% im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 2 und 13.

Beispiel B

Erysiphe-Test (Gerste)/protektiv

Lösungsmittel: 100 Gewichtsteile Dimethylformamid

Emulgator: 0,25 Gewichtsteile Alkylarylpolyglykolether

Zum Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeutchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 11, 8, 1, 9, 2, 13, 12, 17, 19, 20, 21 und 22.

Beispiel C

Cochliobolus sativus-Test (Gerste)/protektiv

Lösungsmittel: 100 Gewichtsteile Dimethylformamid

Emulgator: 0,25 Gewichtsteile Alkylarylpolyglykolether

Zu Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100% rel. Luftfeuchtigkeit in einer Inkubationskabine.

Die Pflanzen werden in einem Gewächshaus bei einer Temperatur von ca. 20° und einer relativen Luftfeuchtigkeit von ca. 80% aufgestellt.

7 Tage nach der Inokulation erfolgt die Auswertung.

Eine deutliche Ueberlegeheit in der Wirksamkeit gegenüber dem Stand der Technik zeigen bei diesem Test z.B. die Verbindungen gemäß folgender Herstellungsbeispiele: 2, 12, 19, 20, 21 und 22.

Beispiel D

Wuchsbeeinflussung bei Zuckerrüben

Lösungsmittel: 30 Gewichtsteile Dimethylformamid

Emulgator: 1 Gewichtsteile Polyoxyethylen-Sorbitan-Monolaurat

Zur Herstellung einer zweckmäßigen Wirkstoffzubereitung vermischt man 1 Gewichtsteil Wirkstoff mit

den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit der Wirkstoffzubereitung besprüht. Nach 14 Tagen Wird der Zuwachs der Pflanzen gemessen und die Wuchsbeeinflussung in Prozent des Zuwachses der Kontrollpflanzen berechnet. Es bedeuten 0% Wuchsbeeinflussung ein Wachstum entsprechend dem der Kontrollpflanzen. Negative Werte kennzeichnen eine Wuchshemmung, positive eine Wuchsförderung gegenüber den Kontrollpflanzen.

Die erfindungsgemäßen Wirkstoffe 8, 12, 1, 9, 13, 19, 20, 21 und 22 zeigen in diesem Test eine bessere Wuchsbeeinflussung als die aus dem Stand der Technik bekannten Verbindungen (B), (D), (E) und (F).

Beispiel E

Wuchshemmung bei Baumwolle

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächschaus bis zur vollen Entfaltung des 5 Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchschemmung in Prozent des Zuwachses der Kontrolle berechnet. Es bedeuten 100% Wuchschemmung den stillstand des Wachstums und 0% ein Wachstum entsprechend dem der Kontrollpflanzen.

Die erfindungsgemäßen Wirkstoffe 8 12, 2, 13, 19, 20, 21 und 22 zeigen in diesem Test eine bessere Wuchshemmung als die aus dem Stand der Technik bekannten Verbindungen (B), (D), (F), (G) und (H).

Beispiel F

Wuchshemmung bei Sojabohnen

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächschaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemäßen Wirkstoffe 8, 12, 1, 2, 13, 19, 20, 21 und 22 zeigen in diesem Test eine bessere Wuchshemmung als die aus dem Stand der Technik bekannten Verbindungen (B), (D), (E), (F) und (G).

Beispiel G

Stimulierung der $CO_2$-Fixierung bei Sojabohnen

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Forlgeblattes angezogen. In diesem Stadium werden die Pflanzen tropfnaß mit den Wirkstoffzubereitungen besprüht. Im weiteren Verlauf des Experimentes wird die $CO_2$-Fixierung der Pflanzen mit üblichen Methoden gemessen. Die Werte werden mit denen nicht mit Wirkstoffen behandelter Kontrollpflanzen verglichen.

Weitere Versuchsdaten und die Resultate dieses Versuches gehen aus der hachfolgenden Tabelle hervor. Es bedeuten:

– Hemmung der —$CO_2$-Fixierung
O $CO_2$-Fixierung wie bei Kontrolle
\+ geringe Stimulierung der $CO_2$-Fixierung
++ starke Stimulierung der $CO_2$-Fixierung
+++ sehr starke Stimulierung der $CO_2$-Fixierung

In diesem Test zeigen die erfindungsgemäßen Wirkstoffe 18, 1 und 13 eine bessere Stimulierung der $CO_2$-Fixierung als die aus dem Stand der Technik bekannten Verbindungen (C), (D), (E), (G) und (H).

## Patentansprüche

1. Substituierte Azolylketone und -alkohole der allgemeinen Formel

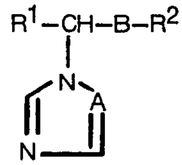

(I)

in welcher

A für ein Stickstoffatom oder die CH-Gruppe steht,

B für die Keto- oder CH(OH)-Gruppe steht,

$R^1$ für geradkettiges oder verzweigtes Alkyl mit 1 bis 12 Kohlenstoffatomen, für geradkettiges oder verzweigtes Alkinyl mit 2 bis 12 Kohlenstoffatomen, oder für gegebenenfalls einfach bis dreifach, gleich oder verschieden substituiertes Phenylalkyl mit 1 bis 4 Kohlenstoffatomen im Alkylteil steht, wobei als Phenylsubsttituenten genannt seien: Halogen; Alkyl, Alkoxy und Alkylthio mit jeweils 1 bis 4 Kohlenstoffatomen; Cyclohexyl; Dialkylamino mit 1 bis 4 Kohlenstoffatomen in jedem Alkylteil; Halogenalkyl, Halogenalkoxy und Halogenalkylthio mit jeweils 1 bis 4 Kohlenstoffatomen und 1 bis 5 gleichen oder verschiedenen Halogenatomen; Nitro; Cyano; Alkoxycarbonyl mit 1 bis 4 Kohlenstoffatomen im Alkoxyteil; sowie gegebenenfalls durch Halogen substituiertes Phenyl und Phenoxy; und ferner

$R^1$ für jeweils gegebenenfalls einfach bis dreifach, gleich oder verschieden durch Alkyl mit 1 bis 4 Kohlenstoffatomen substituiertes Cycloalkyl und Cycloalkylalkyl mit jeweils 3 bis 7 Kohlenstoffatomen im Cycloalkylteil und 1 bis 4 Kohlenstoffatomen im Alkylteil steht;

$R^2$ für einfach bis dreifach, gleich oder verschieden durch Alkyl mit 1 bis 4 Kohlenstoffatomen substituiertes Cycloalkyl mit 3 bis 7 Kohlenstoffatomen, sowie für die Gruppierung $—C(CH_3)_2R^3$ steht, wobei

$R^3$ für geradkettiges oder verzweigtes Alkyl mit 3 bis 6 Kohlenstoffatomen, für geradkettiges oder verzweigtes Alkenyl mit 2 bis 4 Kohlenstoffatomen, für Alkinylmit 3 bis 5 Kohlenstoffatomen oder die —CH=O-Gruppe, Alkoxyiminomethyl mit 1 bis 4 Kohlenstoffatomen im Alkoxyteil, Dialkoxymethyl mit 1 bis 4 Kohlenstoffatomen in jedem Alkoxyteil, Dioxolanyl und Dioxanyl steht,

sowie deren Säureaddtions-Salze und Metallsalz-Komplexe.

2. Verbindungen der allgemeinen Formel I in Anspruch 1, wobei

$R^1$ für geradkettiges oder verzweigtes Alkyl mit 1 bis 6 Kohlenstoffatomen, für geradkettiges oder verzweigtes Alkinyl mit 2 bis 6 Kohlenstoffatomen, sowie für gegebenenfalls einfach bis zweifach, gleich oder verschieden am Phenylteil durch Fluor, Chlor, Methyl, Ethyl, Isopropyl, tert.-Butyl, Methoxy, Methylthio, Cyclohexyl, Dimethylamino, Trifluormethyl, Trifluormethoxy, Trifluormethylthio, Nitro, Cyano, Methoxycarbonyl, oder jeweils gegebenenfalls durch Chlor und Fluor substituiertes Phenyl und Phenoxy substituiertes Phenylalkyl mit 1 bis 2 Kohlenstoffatomen in Alkylteil steht, ferner für jeweils gegebenenfalls einfach oder zweifach, gleich oder verschieden durch Methyl, Ethyl, Isopropyl oder tert.-Butyl substituiertes Cyclopropyl, Cyclopropylmethyl, Cyclopentyl, Cyclopentylmethyl, Cyclohexyl, Cyclohexylmethyl und Cycloheptyl steht;

$R^2$ für jeweils einfach oder zweifach, gleich oder verschieden durch Methyl, Ethyl, Isopropyl oder tert.-Butyl substituiertes Cyclopropyl, Cyclopentyl und Cyclohexylsteht, sowie für die Gruppierung $—(CH_3)_2R^3$, wobei

$R^3$ für geradkettiges oder verzweigtes Alkyl mit 3 bis 6 Kohlenstoffatomen, für Vinyl, Propargyl oder die —CH=O-Gruppe, Methoxyiminomethyl, Dimethoxymethyl, den Dioxolan- und 1,3-Dioxan-Rest steht; und

A und B für die in Anspruch 1 angegebene Bedeutung stehen.

3. Verfahren zur Herstellung von substituierten Azolyl-ketonen und -alkoholen der allgemeinen Formel

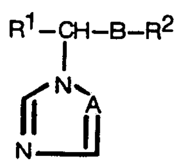

(I)

in welcher

A für ein Stickstoffatom oder die CH-Gruppe steht,

B für die Keto- oder CH(OH)-Gruppe steht,

$R^1$ für geradkettiges oder verzweigtes Alkyl mit 1 bis 12 Kohlenstoffatomen, für geradkettiges oder verzweigtes Alkinyl mit 2 bis 12 Kohlenstoffatomen, oder für gegebenenfalls einfach bis dreifach, gleich oder verschieden substituiertes Phenylalkyl mit 1 bis 4 Kohlenstoffatomen im Alkylteil steht, wobei als

Phenylsubstituenten genannt seien: Halogen; Alkyl, Alkoxy und Alkylthio mit jeweils 1 bis 4 Kohlenstoffatomen; Cyclohexyl; Dialkylamino mit 1 bis 4 Kohlenstofatomen in jedem Alkylteil; Halogenalkyl, Halogenalkoxy und Halogenalkylthio mit jeweils 1 bis 4 Kohlenstoffatomen und 1 bis 5 gleichen oder verschiedenen Halogenatomen; Nitro; Cyano; Alkoxycarbonyl mit 1 bis 4 Kohlenstoffatomen im Alkoxyteil; sowie gegebenenfalls durch Halogen substituiertes Phenyl und Phenoxy; und ferner

$R^1$ für jeweils gegebenenfalls einfach bis dreifach, gleich oder verschieden durch Alkyl mit 1 bis 4 Kohlenstoffatomen substituiertes Cycloalkyl und Cycloalkylalkyl mit jeweils 3 bis 7 Kohlenstoffatomen im Cycloalkylteil und 1 bis 4 Kohlenstoffatomen im Alkylteil steht;

$R^2$ für einfach bis dreifach, gleich oder verschieden durch Alkyl mit 1 bis 4 Kohlenstoffatomen substituiertes Cycloalkyl mit 3 bis 7 Kohlenstoffatomen, sowie für die Gruppierung $—C(CH_3)_2R^3$ steht, wobei

$R^3$ für geradkettiges oder verzweigtes Alkyl mit 3 bis 6 Kohlenstoffatomen, für geradkettiges oder verzweigtes Alkenyl mit 2 bis 4 Kohlenstoffatomen, für Alkinyl mit 3 bis 5 Kohlenstoffatomen oder die —CH=O-Gruppe, Alkoxyiminomethyl mit 1 bis 4 Kohlenstoffatomen im Alkoxyteil, Dialkoxymethyl mit 1 bis 4 Kohlenstoffatomen in jedem Alkoxyteil, Dioxolanyl und Dioxanyl steht,

sowie von dern Säureadditions-Salzen und Metallsalz-Komplexen, dadurch gekennzeichnet, daß man

a) Azolylketone der Formel

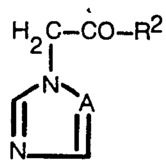

(II)

in welcher

A und $R^2$ die oben angegebene Bedeutung haben, mit einem Alkylierungsmittel der Formel

$$R^1—Z \qquad (III)$$

in welcher

$R^1$ die oben angegebene Bedeutung hat und

Z für eine elektronenanziehende Abgangsgruppierung steht,

in Gegenwart einer Base und in Gegenwart eines organischen Verdünnungsmittels, oder in einem wäßrigorganischen Zweiphasensystem in Gegenwart eines Phasentransferkatalysators, umsetzt; oder

b) Halogenketone der Formel

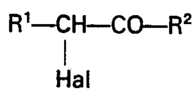

(IV)

in welcher

$R^1$ und $R^2$ die oben angegebene Bedeutung haben und

Hal für Chlor oder Brom steht,

mit Azolen der Formel

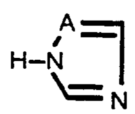

(V)

in welcher

A die oben angegebene Bedeutung hat,

in Gegenwart eines Verdünnungsmittels und in Gegenwart eines Säurebindemittels umsetzt; und gegebenenfalls

c) die nach den Verfahren (a) und (b) erhaltenen Verbindungen der Formel

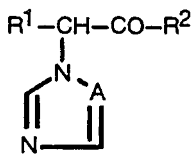

(Ia)

in welcher

A, $R^1$ und $R^2$ die oben angegebene Bedeutung haben,

nach bekannten Methoden in üblicher Weise reduziert und gegebenenfalls noch an die erhaltenen Verbindung der Formel I anschließend eine Säure oder ein Metallsalz addiert.

4. Pflanzenschutzmittel, gekennzeichnet durch einen Gehalt an mindestns einem substituierten Azolylketon oder -alkohol der Formel (I) in Anspruch 1 bzw. an einem Säureadditions-Salz oder Metallsalz-Komplex eines Stoffes der Formel (I).

5. Fungizide und das Pflanzenwachstum regulierende Mittel, gekennzeichnet durch einen Gehalt an mindestens einem substituierten Azolylketon oder -alkohol der Formel (I) in Anspruch 1 bzw. an einem Säureadditions-Salz oder Metallsalz-Komplex eines Stoffes der Formel (I).

6. Verfahren zur Bekämpfung von Pilzen und zur Regulierung des Pflanzenwachstums, dadurch gekennzeichnet, daß man substituierte Azolylketone oder -alkohole der Formel (I) in Anspruch 1 bzw. deren Säureadditions-Salze oder Metallsalz-Komplexe auf Pilze, Pflanzen oder ihren Lebensraum einwirken läßt.

7. Verwendung von substituierten Azolylketonen und -alkoholen der Formel (I) in Anspruch 1 bzw. von deren Säureadditions-Salzen oder Metallsalz-Komplexen als Pflanzenschutzmittel.

8. Verwendung von substituierten Azolylketonen und -alkoholen der Formel (I) in Anspruch 1 bzw. von deren Säureadditions-Salzen oder Metallsalz-Komplexen zur Bekämpfung von Pilzen und Regulierung des Pflanzenwachstums.

9. Verfahren zur Herstellung von fungiziden und das Wachstum von Pflanzen regulierenden Mitteln, dadurch gekennzeichnet, daß man substituierte Azolylketone und -alkohole der Formel (I) in Anspruch 1 bzw. deren Säureadditions-Salze oder Metallsalz-Komplexe mit Streckmitteln und/oder oberflächenaktiven Mitteln vermischt.

10. Azolylketone der allgemeinen Formel

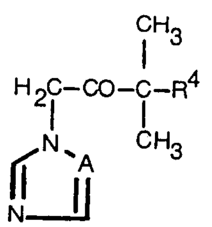

(IIa)

in welcher

A für ein Stickstoffatom oder die CH-Gruppe steht und

$R^4$ für die CHO-Gruppe Alkoximinomethyl mit 1 bis 4 Kohlenstoffatomen im Alkoxyteil, Dialkoxymethyl mit 1 bis 4 Kohlenstoffatomen in jedem Alkoxyteil sowie für Dioxolanyl und Dioxanyl steht.

**Revendications**

1. Azolylcétones et azolylalcools substitués de formule générale

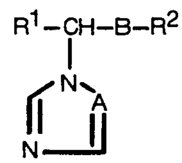

(I)

dans laquelle

A désigne un atome d'azote ou le groupe CH,

B est le groupe céto ou CH(OH),

$R^1$ est un groupe alkyle ä chaîne droite ou à chaîne ramifiée ayant 1 à 12 atomes de carbone, un groupe alcynyle à chaîne droite ou à chaîne ramifiée de 2 à 12 atomes de carbone ou un groupe phénylalkyle portant éventuellement 1 à 3 substituants identiques ou différents et ayant 1 à 4 atomes de carbone dans la partie alkyle, avec comme substituants du groupe phényle: un halogène; des groupes alkyle alkoxy et alkylthio ayant chacun 1 à 4 atomes de carbone; un groupe cyclohexyle; un groupe dialkylamino ayant 1 à 4 atomes de carbone dans chaque partie alkyle; des groupes halogénalkyle, halogénalkoxy et halogénalkylthio ayant chacun 1 à 4 atomes de carbone et 1 à 5 atomes d'halogènes identiques ou différents; un groupe nitro; cyano; alkoxycarbonyle ayant 1 à 4 atomes de carbone dans la partie alkoxy; ainsi que le cas échéant un groupe phényle et un groupe phénoxy substitués par un halogène; en outre

$R^1$ représente un groupe cycloalkyle et un groupe cycloalkylalkyle portant chacun le cas échéant 1 à 3 substituants alkyle de 1 à 4 atomes de carbone identiques ou différents, avec chacun 3 à 7 atomes de carbone dans la partie cycloalkyle et 1 à 4 atomes de carbone dans la partie alkyle;

$R^2$ désigne un groupe cycloalkyle de 3 à 7 atomes de carbone portant 1 à 3 substituants alkyle de 1 à 4 atomes de carbone identiques ou différents, ainsi que le groupement $-C(CH_3)_2R^3$, dans lequel

$R^3$ est un groupe alkyle à chaîne droite ou à chaîne ramifiée de 3 à 6 atomes de carbone, un groupe alcényle à chaîne droite ou à chaîne ramifiée de 2 à 4 atomes de carbone, un groupe alcynyle de 3 à 5 atomes de carbone ou le groupe $-CH=O$, un groupe alkoxyiminométhyle de 1 à 4 atomes de carbone dans

la partie alkoxy, un groupe dialkoxyméthyle de 1 à 4 atomes de carbone dans chaque partie alkoxy, un groupe dioxolannyle et un groupe dioxannyle,
ainsi que leurs sels d'addition d'acides et leurs complexes de sels métalliques.

2. Composés de formule générale I suivant la revendication 1, dans laquelle

$R^1$ est un groupe alkyle à chaîne droite ou à chaîne ramifiée ayant 1 à 6 atomes de carbone, un groupe alcynyle à chaîne droite ou à chaîne ramifiée ayant 2 à 6 atomes de carbone, un groupe phénylalkyle portant éventuellement 1 ou 2 substituants identiques ou différents, ayant 1 ou 2 atomes de carbone dans la partie alkyle, les substituants du groupe phényle pouvant être: le fluor, le chlore, un groupe méthyle, éthyle, isopropyle, tertiobutyle, méthoxy, méthylthio, cyclohexyle, diméthylamino, trifluorométhyle, trifluorométhoxy, trifluorométhylthio, nitro, cyano, méthoxycarbonyle, ou un groupe phényle et un groupe phénoxy substitués chacun le cas échéant par du chlore et du fluor; en outre des groupes cyclopropyle, cyclopropylméthyle, cyclopentyle, cyclopentylméthyle, cyclohexyle, cyclohexylméthyle et cycloheptyle portant chacun le cas échéant 1 ou 2 substituants méthyle, éthyle, isopropyle ou tertiobutyle identiques ou différents;

$R^2$ désigne des groupes cyclopropyle, cyclopentyle et cyclohexyle portant chacun 1 ou 2 substituants méthyle, éthyle, isopropyle ou tertiobutyle identiques ou différents, ainsi que le groupement $—(CH_3)_2R^3$, dans lequel

$R^3$désigne un groupe alkyle à chaîne droite ou à chaîne ramifiée de 3 à 6 atomes de carbone, un groupe vinyle, propargyle ou le groupe —CH=O, méthoxyiminométhyle, diméthoxyméthyle, le reste dioxolanne et le reste 1,3-dioxanne

et A et B ont la définition indiquée dans la revendication 1.

3. Procédé de production d'azolycétones et d'azolylalcools substitués de formule générale

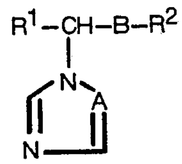

(I)

dans laquelle

A désigne un atome d'azote ou le groupe CH,

B est le groupe céto ou CH(OH),

$R^1$ est un groupe alkyle à chaîne droite ou à chaîne ramifiée ayant 1 à 12 atomes de carbone, un groupe alcynyle à chaîne droite ou à chaîne ramifiée de 2 à 12 atomes de carbone ou un groupe phénylalkyle portant éventuellement 1 à 3 substituants identiques ou différents et ayant 1 à 4 atomes de carbone dans la partie alkyle, avec comme substituants du groupe phényle: un halogène; des groupes alkyle, alkoxy et alkylthio ayant chacun 1 à 4 atomes de carbone; un groupe cycloalkyle; un groupe dialkylamino ayant 1 à 4 atomes de carbone dans chaque partie alkyle; des groupes halogénalkyle, halogénalkoxy et halogénalkylthio ayant chacun 1 à 4 atoms de carbone et 1 à 5 atomes d'halogènes identiques ou différents; un groupe nitro; cyano; alkoxycarbonyle ayant 1 à 4 atomes de carbone dans la partie alkoxy; ainsi que le cas échéant un groupe phényle et un groupe phénoxy substitués par un halogène; en outre

$R^1$ représente un groupe cycloalkyle et un groupe cycloalkylalkyle portant chacun le cas échéant 1 à 3 substituants alkyle de 1 à 4 atomes de carbone identiques ou différents, avec chacun 3 à 7 atomes de carbone dans la partie cycloalkyle et 1 à 4 atomes de carbone dans la partie alkyle;

$R^2$ désigne un groupe cycloalkyle de 3 à 7 atomes de carbone portant 1 à 3 substituants alkyle de 1 à 4 atomes de carbone identiques ou différents, ainsi que le groupement $—C(CH_3)_2R^3$, dans lequel

$R^3$ est un groupe alkyle à chaîne droite ou à chaîne ramifiée de 3 à 6 atomes de carbone, un groupe alcényle à chaîne droite ou à chaîne ramifiée de 2 à 4 atomes de carbone, un groupe alcynyle de 3 à 5 atomes de carbone ou le groupe —CH=O, un groupe alkoxyiminométhyle de 1 à 4 atomes de carbone dans la partie alkoxy, un groupe dialkoxyméthyle de 1 à 4 atomes de carbone dans chaque partie alkoxy, un groupe dioxolannyle et un groupe dioxannyle,

ainsi que leurs sels d'addition d'acides et leurs complexes de sels métalliques, caractérisé en ce que:

a) on fait réagir des azolylcétones de formule

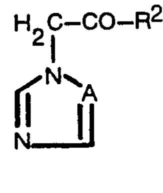

(II)

dans laquelle

A et $R^2$ ont la définition indiquée ci-dessus, avec un agent d'alkylation de formule

$$R^1 - Z \qquad (III)$$

dans laquelle

$R^1$ a la définition indiquée ci-dessus et

Z est un groupe partant attirant les électrons,

en présence d'une base et en présence d'un diluant organique, ou dans un système formé d'une phase aqueuse et d'une phase organique en présence d'un catalyseur de transfert de phase; ou bien

b) on fait réagir des halogénocétones de formule

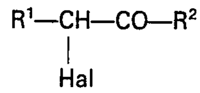

(IV)

dans laquelle

$R^1$ et $R^2$ ont la définition indiquée ci-dessus et Hal désigne le chlore ou le brome, avec des azoles de formule

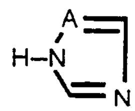

(V)

dans laquelle

A a la définition indiquée ci-dessus,

en présence d'un diluant et en présence d'un accepteur d'acide; et le cas échéant

c) on réduit de manière classique par des procédés connus les composés obtenus par les procédés (a) et (b), de formule

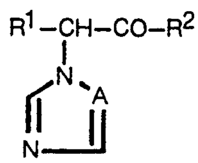

(Ia)

dans laquelle

A, $R^1$ et $R^2$ ont la définition indiquée ci-dessus, puis le cas échéant, on additionne encore un acide ou un sel métallique sur les composés obtenus de formule I.

4. Composition pour la protection des plantes, caractérisée par une teneur en au moins une azolylcétone ou un azolyalcool substitué de formule (I) suivant la revendication 1 ou en un sel d'addition d'acide ou un complexe de sel métallique d'une composé de formule I.

5. Compositions fongicides et influençant la croissance de végétaux, caractérisées par une teneur en au moins une azolylcétone ou un azolylalcool substitué de formule (I) suivant la revendication 1 ou en un sel d'addition d'acide ou un complexe de sel métallique d'un composé de formule (I).

6. Procédé pour combattre des champignons et pour influencer la croissance de végétaux, caractérisé en ce qu'on fait agir des azolylcétones ou des azolylalcools substitués de formule (I) suivant la revendication 1 ou leurs sels d'addition d'acides ou leurs complexes de sels métalliques sur des champignons, des plantes ou leur milieu.

7. Utilisation d'azolylcétones et d'azolylalcools substitués de formule (I) suivant la revendication 1 ou de leurs sels d'addition d'acides ou de leurs complexes de sels métalliques comme agents de protection des plantes.

8. Utilisation d'azolylcétones et d'azolylalcools substitués de formule (I) suivant la revendication 1 ou de leurs sels d'addition d'acides ou de leurs complexes de sels métalliques pour combattre des champignons et pour influencer la croissance de végétaux.

9. Procédé de préparation de compositons fongicides et influençant la croissance de végétaux, caractérisé en ce qu'on mélange des azolylcétones et des azolylalcools substitués de formule (I) suivant la revendication 1 ou leurs sels d'addition d'acides ou leurs complexes de sels métalliques, avec des diluants et/ou des agents tensioactifs.

10. Azolylcétones de formule générale

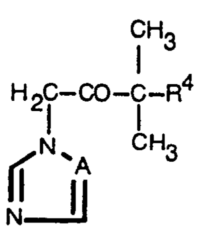

(IIa)

dans laquelle

A désigne un atome d'azote ou le groupe CH et

$R^4$ est un groupe CHO, alkoxyiminométhyle ayant 1 à 4 atomes de carbone dans la partie alkoxy, dialkoxyméthyle ayant 1 à 4 atomes de carbone dans chaque partie alkoxy, ainsi que dioxolannyle et dioxannyle.

**Claims**

1. Substituted azolyl-ketones and -alcohols of the general formula

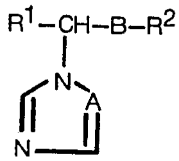

(I)

in which

A represents a nitrogen atom or the CH group,

B represents the keto or CH(OH) group,

$R^1$ represents straight-chain or branched alkyl having 1 to 12 carbon atoms, straight-chain or branched alkinyl having 2 to 12 carbon atoms, or phenylalkyl which has 1 to 4 carbon atoms in the alkyl part and is optionally monosubstituted to trisubstituted by identical or different substituents, the following being mentioned as phenyl substituents: halogen; alkyl, alkoxy and alkylthio, each having 1 to 4 carbon atoms; cyclohexyl; dialkylamino having 1 to 4 carbon atoms in each alkyl part; halogenoalkyl, halogenoalkoxy and halogenoalkylthio, each having 1 to 4 carbon atoms and 1 to 5 identical or different halogen atoms; nitro; cyano; alkoxycarbonyl having 1 to 4 carbon atoms in the alkoxy part; and optionally halogen-substituted phenyl and phenoxy; and

$R^1$ also represents cycloalkyl and cycloalkylalkyl, each of which has 3 to 7 carbon atoms in the cycloalkyl part and 1 to 4 carbon atoms in the alkyl part and each of which is optionally monosubstituted to trisubstituted by identical or different alkyl radicals having 1 to 4 carbon atoms;

$R^2$ represents cycloalkyl which has 3 to 7 carbon atoms and is monosubstituted to trisubstituted by identical or different alkyl radicals having 1 to 4 carbon atoms and also represent the grouping $—C(CH_3)_2R^3$, wherein

$R^3$ represents straight-chain or branched alkyl having 3 to 6 carbon atoms, straight-chain or branched alkenyl having 2 to 4 carbon atoms, alkinyl having 3 to 5 carbon atoms or the $—CH{=}O$ group, alkoxyiminomethyl having 1 to 4 carbon atoms in the alkoxy part, dialkoxymethyl having 1 to 4 carbon atoms in each alkoxy part, and dioxolanyl and dioxanyl,

and their acid addition salts and metal salt complexes.

2. Compounds of the general formula I in Claim 1, wherein

$R^1$ represents straight-chain or branched alkyl having 1 to 6 carbon atoms, straight-chain or branched alkinyl having 2 to 6 carbon atoms, and phenylalkyl which has 1 to 2 carbon atoms in the alkyl part and is optionally monosubstituted or disubstituted in the phenyl part by identical or different substituents from amongst fluorine, chlorine, methyl, ethyl, isopropyl, tert.-butyl, methoxy, methylthio, cyclohexyl, dimethylamino, trifluoromethyl, trifluoromethoxy, trifluoromethylthio, nitro, cyano, methoxycarbonyl, or phenyl and phenoxy, each of which is optionally substituted by chlorine and fluorine, or represents cyclopropyl, cyclopropylmethyl, cyclopentyl, cyclopentylmethyl, cyclohexyl, cyclohexylmethyl and cycloheptyl, each of which is optionally monosubstituted or disubstituted by identical or different substituents from amongst methyl, ethyl, isopropyl or tert.-butyl;

$R^2$ represents cyclopropyl, cyclopentyl and cyclohexyl, each of which is optionally monosubstituted or disubstituted by identical or different substituents from amongst methyl, ethyl, isopropyl and tert.-butyl, and represents the grouping $—(CH_3)_2R^3$, wherein

$R^3$ represents straight-chain or branched alkyl having 3 to 6 carbon atoms, vinyl, propargyl or the $—CH{=}O$ group, methoxyiminomethyl, dimethyloxymethyl, or the dioxolane and 1,3-dioxane radicals, and

A and B have the meaning given in Claim 1.

3. Process for the preparation of substituted azolyl-ketones and -alcohols of the general formula

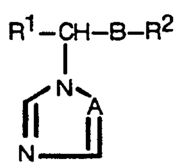

(I)

in which

A represents a nitrogen atom or the CH group,

B represents the keto or CH(OH) group,

$R^1$ represents straight-chain or branched alkyl having 1 to 12 carbon atoms, straight-chain or branched alkinyl having 2 to 12 carbon atoms, or phenylalkyl which has 1 to 4 carbon atoms in the alkyl part and is optionally monosubstituted to trisubstituted by identical or different substituents, the following being mentioned as phenyl substituents: halogen; alkyl, alkoxy and alkylthio, each having 1 to 4 carbon atoms; cyclohexyl; dialkylamino having 1 to 4 carbon atoms in each alkyl part; halogenoalkyl, halogenoalkoxy and halogenoalkylthio, each having 1 to 4 carbon atoms and 1 to 5 identical or different halogen atoms; nitro; cyano; alkoxycarbonyl having 1 to 4 carbon atoms in the alkoxy part; and optionally halogen-substituted phenyl and phenoxy; and

$R^1$ also represents cycloalkyl and cycloalkylalkyl, each of which has 3 to 7 carbon atoms in the cycloalkyl part and 1 to 4 carbon atoms in the alkyl part and each of which is optionally monosubstituted to trisubstituted by identical or different alkyl radicals having 1 to 4 carbon atoms;

$R^2$ represents cycloalkyl which has 3 to 7 carbon atoms and is monosubstituted to trisubstituted by identical or different alkyl radicals having 1 to 4 carbon atoms and also represents the grouping $-C(CH_3)_2R^3$, wherein

$R^3$ represents straight-chain or branched alkyl having 3 to 6 carbon atoms, straight-chain or branched alkenyl having 2 to 4 carbon atoms, alkinyl having 3 to 5 carbon atoms or the $-CH{=}O$ group, alkoxyiminomethyl having 1 to 4 carbon atoms in the alkoxy part, dialkoxymethyl having 1 to 4 carbon atoms in each alkoxy part, and dioxolanyl and dioxanyl,

and their acid addition salts and metal salt complexes, characterised in that

a) azolyl-ketones of the formula

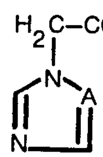

(II)

in which

A and $R^2$ have the meaning given above,

are reacted with an alkylating agent of the formula

$$R^1 - Z \qquad (III)$$

in which

$R^1$ has the meaning given above and

Z represents an electron-attracting leaving grouping,

in the presence of a base and in the presence of an organic diluent, or in an aqueous-organic two-phase system in the presence of a phase-transfer catalyst; or

b) halogenoketones of the formula

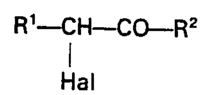

(IV)

in which

$R^1$ and $R^2$ have the meaning given above and

Hal represents chlorine or bromine,

are reacted with azoles of the formula

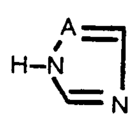

(V)

in which

A has the meaning given above,

in the presence of a diluent and in the presence of an acid-binding agent; and, if appropriate,

c) the compounds obtained by processes (a) and (b), of the formula

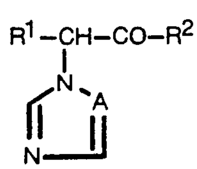

(Ia)

in which

A, $R^1$ and $R^2$ have the meaning given above, are reduced in a customary manner, according to known methods, and, if appropriate, the resulting compounds of the formula (I) are then subjected to an addition reaction with an acid or a metal salt.

4. Plant protection agents, characterized in that they contain at least one substituted azolyl-ketone or -alcohol of the formula I in Claim 1 or an acid addition salt or a metal salt complex of a substance of the formula (I).

5. Fungicides and plant growth-regulating agents, characterized in that they contain at least one substituted azolyl-ketone or -alcohol of the formula (I) in Claim 1 or an acid addition salt or a metal salt complex of a substance of the formula (I).

6. Process for combating fungi and for regulating plant growth, characterized in that substituted azolyl-ketones or -alcohols of the formula (I) in Claim 1 or their acid addition salts or metal salt complexes are allowed to act on fungi, plants or their habitat.

7. Use of substituted azolyl-ketones and -alcohols of the formula (I) in Claim 1 or their acid addition salts or metal salt complexes, as plant protection agents.

8. Use of substituted azolyl-ketones and -alcohols of the formula (I) in Claim 1 or of their acid addition salts or metal salt complexes, for combating fungi and regulating plant growth.

9. Process for the preparation of fungicidal agents and plant growth-regulating agents, characterized in that substituted azolyl-ketones and -alcohols of the formula (I) in Claim 1 or their acid addition salts or metal salt complexes are mixed with extenders and/or surface-active agents.

10. Azolyl-ketones of the general formula

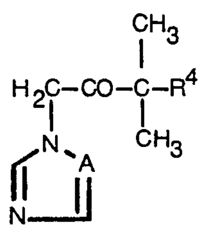

(IIa)

A represents a nitrogen atom or the CH group, and

$R^4$ represents the CHO group alkoximinomethyl having 1 to 4 carbon atoms in the alkoxy part, dialkoxymethyl having 1 to 4 carbon atoms in each alkoxy part or dioxolanyl and dioxanyl.